# EUROPEAN PATENT APPLICATION

(11) **EP 1 533 385 A1**
(43) Date of publication of application: **25.05.2005**
(21) Application number: 04077474.7
(22) Date of filing: 06.09.2004
(51) Int. Cl.: C12Q 1/68, C07H 21/00

(54) **Templated compounds for generation of encoded molecules and directional methods using such compounds**

(30) Priority: 05.09.2003 DK 200301276; 05.09.2003 US 500265 P
(71) Applicant: Nuevolution A/S, 2100 Copenhagen 0 (DK)
(72) Inventor: Glad, Sanne Schroder, 2750 Ballerup (DK); Slok, Frank Abilgaard, 3450 Allerod (DK)
(74) Representative: HOEIBERG A/S

(57) **Abstract**

Disclosed is a compound comprising a bifunctional chemical entity attached to a nucleoside derivative and a directing element capable of positioning the bifunctional chemical entity in a predominate direction. The compound is useful in the formation of encoded molecules by contacting a template nucleic acid with one or a plurality of the compounds under conditions that provide for the formation of a complementary template, and reacting the bifunctional chemical entities to form an encoded molecule

## Description

### Technical Field of the Invention

The present invention relates to compounds comprising a bifunctional chemical entity, which during formation of an encoded molecule, is positioned in a predominate direction. The positioning in a preferred direction of the bifunctional chemical entity may entail among other things that a full length encoded molecule is formed. The present invention also relates to nucleotide analogues that are substrates for a polymerase and to a double stranded nucleic acid comprising the compounds of the invention.

### Background of the Invention

Biological systems allow for the synthesis of α-peptides by a process known as translation. The natural translation process requires a mRNA template and a plurality of charged tRNA building blocks. A ribosome is initially attached to the mRNA template and directs the recognition between anti-codons of tRNA building blocks and complementing codons of the mRNA template. Concurrently with the recognition process a α-amino acid residue of the tRNA is reacted with a nascent polypeptide to extent nascent polypeptide with a monomer unit.

Recently, a method has been suggested in WO 02/103008 A2 (the content of which being incorporated herein in its entirety by reference) for producing other encoded molecules than α-peptides. According to one embodiment of this publication, a nucleic acid template and a plurality of nucleoside derivative building blocks carrying a functional entity is provided. Using the nucleic acid template, complementing nucleoside derivatives are incorporated into a complementary strand. Subsequent to, or simultaneous with the formation of the complementing template, the functional entities are reacted to form an encoded molecule. The end product of the process is a bifunctional complex comprising an encoded molecule attached to the complementing template.

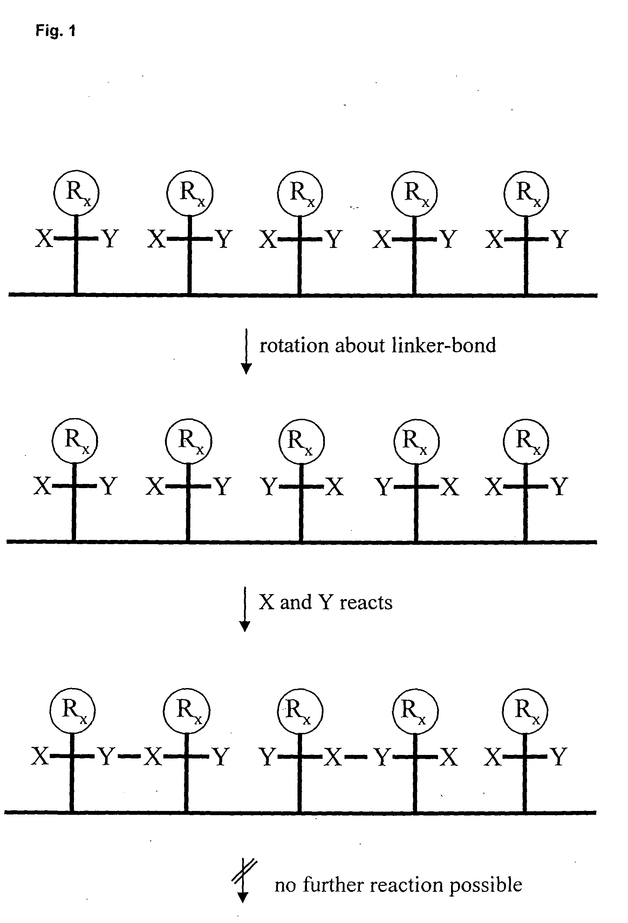

The present invention relates to certain aspects of the above publication, especially aspects in which a bifunctional chemical entity is attached to a nucleoside derivative, as a special situation arises when employing bifunctional chemical entities due to a potential free rotation around the linker-nucleotide bond. As illustrated in Fig. 1, a bifunctional chemical entity bears two different reactive groups 'X' and 'Y', e.g. both a nucleophile and an electrophile, where 'X' on one chemical entity is meant to react with 'Y' on the neighbour chemical entity, either directly or through a cross-linking agent. If all linker-chemical entity units orient identically with respect to the parent nucleotide, directional polymerization will take place and a complete product of say 5 units will be formed. However, rotation around the linker-bond of some, but not all, linker-chemical entities so that the relative orientation of the two functionalities reverses leads to a clustering situation, where the reacting groups are arranged so that reaction can take place in two different directions. This unfavourable situation can be avoided by using fixed functional entities thereby preventing rotation around the nucleotide-linker bond. Fixing the chemical entities may be obtained by attaching the chemical entity not by one but by two covalent bonds (i.e. two linkers) to the nucleotide. The additional bond may be formed directly by one of the functionalities, or the two reactive groups may be attached by separate 'arms' on a fixed backbone. In the first situation the additional bond may be broken during the reaction, whereas the additional bond in the latter should be constructed so that also this bond is cleavable after reaction, to release the final product.

The present invention aims at providing compounds which tends to be directed in a predominate direction so that a cluster formation is avoided. In general, directional encoding will lead to full-length products and in addition to products of known polarity. Thus an unambiguous relationship between the genetic information of the template and the encoded molecule may be obtained.

### Summary of the Invention

The present invention concerns a compound comprising a bifunctional chemical entity attached to a nucleoside derivative and a directing element capable of positioning the bifunctional chemical entity in a predominate direction.

DNA made up of a double helix has an inherent twist of the backbone, positioning one base pair not on top of the previous but shifted by a certain distance and in turn rotated by 36 degrees. This means that the distance between neighbouring attachment points is larger (typically on the order of 4.4 Å) than the vertical distance between two base pairs (3.4 Å). In addition, the rotation results in different starting directions of the linkers resulting in increasing distance between equal atoms of neighbouring stiff linkers.

For bifunctional entities attached to a nucleotide, there are two possibilities of reaction: the functionality placed in the 3' direction of the nucleic acid strand can attack a neighbouring functionality positioned in the 5' direction of the nucleic acid strand or the functionality placed in the 5' direction can attack the neighbouring functionality positioned in the 3' direction of the nucleic acid strand. Due to the twist of DNA these two reactions are geometrically different, and the reaction distance of the 3'-5' reaction is significantly shorter than the reaction distance of the 5'-3' reaction - simply due to the inherent twist of DNA. The present invention takes advantage of the geometry of the DNA double helix, whereby directionality can be obtained without covalently constraining the linkers. By incorporating a directing element in the vicinity of the functional entity the bifunctional chemical entities will be influenced by the DNA environment. By careful design of linkers this can be utilised for directionality purposes.

Generally, the directing element interacts with one or more major groove atoms or an internucleoside linkage of a double helix nucleic acid. The interaction may be of any appropriate nature, e.g. the directing element is attracted or repulsed by major groove or internucleoside linkage atoms of a nucleic acid double helix. Importantly, the directing element must interact in a way that positions the bifunctional chemical entity in a certain preferred position. The attraction and/or repulsion may involve interaction between single atoms or between groups of atoms. The interaction between single atoms or groups of atoms may be an ionic attraction or repulsion. The interaction between groups of atoms may involve hydrophobic/hydrophilic interaction, van der Waal interaction etc. By preferred or predominate orientation or direction of the bifunctional chemical entity is meant that the bifunctional chemical entity is prone to be positioned in a certain direction in more than 50%, preferably 70% and most preferred 90% of the time. The time a bifunctional entity is in a certain direction may be calculated by evaluating the possibility of each of the possible conformations. In general, the term *conformation* refers to individual structural orientations differing by simple rotation about single bonds. Different conformations may in addition give rise to different overall *configurations,* by which is meant an overall arrangement of bifunctional chemical entities on all modified nucleosides that give rise to one specific direction of reaction. As an example, four bifunctional chemical entities arranged with all 'X's' in the same direction corresponds to one specific configuration, and four bifunctional chemical entities arranged e.g. with two 'X's' pointing in one direction and the two other in the opposite direction corresponds to another specific configuration. Within one configuration many different conformations are possible, but all of these result in the same 'most probable' product since the overall orientation (direction) of reactive groups is preserved.

The directing element may by chosen among a variety of different chemical components. In an aspect of the invention the directing element comprises an optionally substituted 5-, 6-, or 7-membered ring structure. In a preferred aspect the directing element comprises an aromatic or hetero-aromatic ring system. In order to obtain a sufficiently high influence of the directing element on the bifunctional entity, it may be desired to position the bifunctional chemical entity and the directing element within a certain distance. In a preferred aspect of the invention, the extended length between the directing element and the bifunctional chemical entity is 4Å or less. The term extended length means herein the conformation that leads to the longest distance.

The directing element can be positioned relative to the bifunctional chemical entity and the nucleoside derivative in any appropriate way. In one aspect of the invention, the directing element is positioned in the linkage between the nucleoside derivative and the bifunctional chemical entity. In another embodiment the bifunctional chemical entity is positioned between a linkage to the nucleoside derivative and the directing element. In a still further aspect of the invention, the directing element is attached to a linkage connecting the bifunctional chemical entity and the nucleoside derivative.

The functionalities of the bifunctional chemical entities may be chosen within a wide range of reactive groups. In one aspect of the invention, the two functionalities are capable of reacting with each other. Notably, the bifunctional chemical entity comprises a nucleophile and an electrophile as the two functionalities. An example of a nucleophlile is an amine and an example of an electrophile is a carboxylic acid or an ester. In an aspect of the invention, the functionalities are protected by suitable protection groups.

The compound of the invention may be composed solely of the nucleoside derivative, the directing element and the bifunctional chemical entity. However, in some aspects the bifunctional chemical entity is attached to the nucleoside derivative through a spacing element. The spacing element serves a variety of functions when present, the main function being distancing the bifunctional chemical entity from the nucleoside derivative. In one aspect, the spacing element comprises a chemical bond which includes electrons from overlapping p-orbitals. Suitably, the spacing element includes a triple bond, an aromatic- or heteroaromatic ring system, or a hetero atom.

The distance between the bifunctional chemical entity and the nucleoside derivative is suitably chosen such that a suitable reactivity is obtained. In certain aspects, the extended length between the bifunctional chemical entity and the nucleoside is between 3 and 12Å.

The bifunctional chemical entity can be attached to any position of the nucleotide derivative. In a preferred aspect the bifunctional chemical entity is attached through a linker to the nucleobase of the nucleoside. The nucleobase derivative may be a naturally occurring nucleobase or a synthetic nucleobase. In some aspects of the invention, the nucleobase is selected among adenine, 7-deaza-adenine, uracil, guanidine, 7-deaza-guanidine, thymine, and cytosine. In preferred aspects of the invention, the bifunctional chemical entity is attached through a linker to the 5 position of pyrimidine type nucleobases or the 7 or 8 position of purine type nucleobases.

The invention also relates to nucleotide analogues comprising the above nucleotide derivative attached to a bifunctional molecule. The nucleotide analogue may be incoporated into a complementary strand using various means, e.g. chemical ligation or enzymatic polymerisation. Usually it is preferred to use a polymerase or a ligase to incorporate the nucleotide analogue. Therefore, the nucleotide analogue is preferably a substrate for a polymerase or a ligase. An example of a substrate for polymerases is nucleotide triphosphates. The nucleotide is usually a mononucleotide triphosphate but may be an oligonucleotide triphosphate as using the teaching of WO 01/16366, the content of which is incorporated herein by reference. An example of a substrate for a ligase is an oligonucleotide monophosphate.

The invention is furthermore directed to a method for directing the structural orientation of a bifunctional chemical entity, wherein a nucleoside derivative comprising a bifunctional chemical entity further comprises a directing element capable of positioning the bifunctional chemical entity in a predominate direction. In a preferred aspect, the directing element interacts with the major groove atoms or internucleoside linkage of a double helix nucleic acid. More preferred, the directing element comprises an optionally substituted 5-, 6-, or 7-membered ring structure. In some aspects, the directing element comprises an aromatic or hetero-aromatic ring system.

The invention also relates to a method for obtaining an encoded molecule comprising contacting a template nucleic acid with one or a plurality of compounds according to the invention, under conditions that provide for the formation of a complementary template, and reacting the bifunctional chemical entities to form an encoded molecule.

The template comprises a sequence of nucleotides which is complemented by the compounds of the invention and incorporated into a complementary strand. When a polymerase is used for incorporation a primer is usually initially annealed to the template to obtain a site for the polymerase to bind. Subsequently, each of a plurality of nucleotides is incorporated into a complementary strand by extending the primer. In one aspect of the invention, native nucleobases or close analogous are used in the compounds of the invention to obtain a conventional Watson-Crick base pairing scenario, i.e. an A forms a specific base-pair with T and C forms a specific base-pair with G. The specific base-pairing allows the genetic encoding of the bifunctional chemical entity in the final encoded molecule because it is possible to decode the template or alternatively the complementing template to establish the synthesis history of the encoded molecule.

In an aspect of the invention a plurality of templates is provided to produce a library of encoded molecules. The library of encoded molecules has a variety of uses, e.g. as possible ligands to a pharmaceutical interesting target or as a vehicle for carrying a pharmaceutical active substance into a tissue or cell of interest.

The encoded molecule is generally a polymer in the sense that a head-to-tail reaction of the bifunctional chemical entities occurs. It is to be understood that the units of the polymer may be identical or different and the type of reaction may vary over the encoded molecule. Using a single nucleobase in the compound of the invention allows for four different bifunctional chemical entities, if a one-to- one relationship between the genetic information of the nucleobase and the identity of the bifunctional chemical entity is to be maintained. However, using two or more nucleobases in the compound of the invention allows for the formation of a more diverse monomer composition.

The invention also is directed to a double stranded nucleic acid having one or more compounds of the invention incorporated therein. In a preferred aspect the bifunctional chemical entities have been reacted under conditions in which they had a predominate orientation. After the reaction or simultaneously with the reaction, one or more linking moieties may be cleaved to efficiently display the encoded molecule.

### Brief Description of the Figures

Fig. 1 shows a situation in which a cluster is formed due to lack of directionality of the bifunctional chemical entity.
Fig. 2 discloses a general composition of the compound of the invention,
Fig. 3 shows a design of the linker in which the directing element is between a spacing element and a bifunctional entity.
Fig. 4 discloses a design of the linker in which the directing element is attached to the spacing element.
Fig. 5 shows a design of the compound in which the bifunctional chemical entity is between the spacing element and the directing element.
Fig. 6 discloses a predominate position of a bifunctional entity of example 1 relative to the DNA double helix.
Fig. 7 shows a diagram of reaction distances as function of conformation energies for the compound of Example 1.
Fig. 8 depicts two conformations of the compound of example 2 when incorporated into a DNA double helix.
Fig. 9 discloses a diagram of reaction distance as function of conformation energies for the compound of Example 2.

### Detailed Description of the Invention

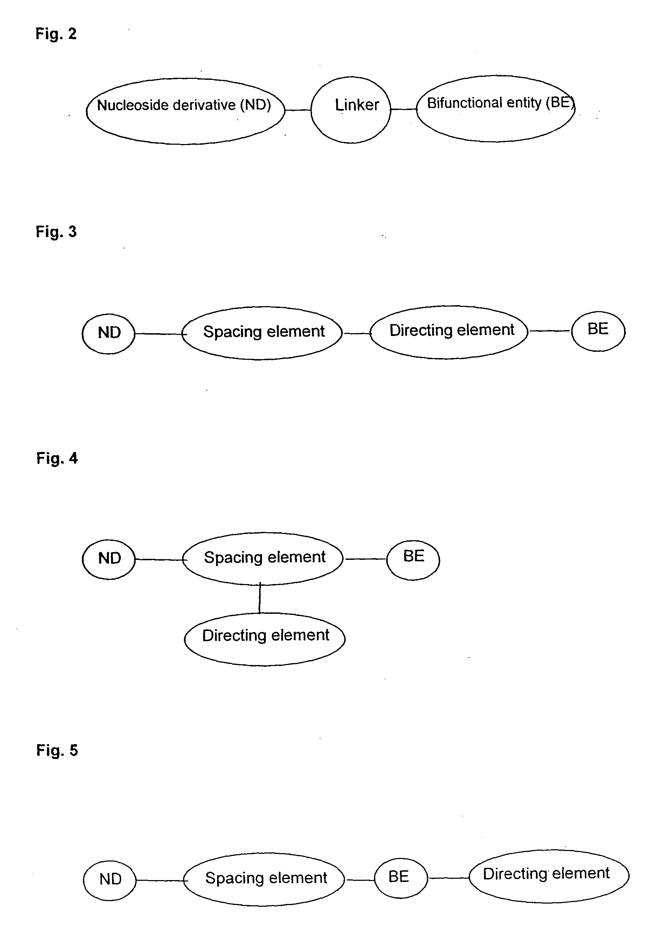

In the discussion of the present invention it is convenient to introduce the term linker, as a residue or chemical bond separating the bifunctional chemical entity (BE) and the nucleoside derivative (ND), see Fig. 2. The linker may or may not include a spacing element and/or the directing element. In a preferred aspect the linker is of a specified length and comprised of two elements: a spacing element (SE) and a directing element (DE), thereby ensuring reaction in the vicinity of the double-stranded DNA (comprised of the template and the linked complementing units) providing a specific overall orientation of bifunctional units and thereby a high degree of directionality of polymerisation.

The directing element can be linking the SE and the bifunctional chemical entity (Fig. 3), the DE can be attached the SE as a substituent so that the SE directly links the nucleoside derivative and the BE (Fig. 4), or alternatively, the DE can be attached at the outer side of the BE (Fig. 5).

The purpose of the spacing element is to ensure some distance between the BE and the nucleotide derivative typically no more than a favourable van-der-Waal distance just excluding water intrusion between the DNA and the bifunctional chemical entity. The SE is preferably comprised of a chemical unit bearing π-electrons, i.e. a triple bond, an aromatic or heteroaromatic ring system, or a heteroatom such as O, S, or N. The minimum length of the spacing element is 0 atoms, i.e. the spacing element is absent. When present, the length of the spacing element may be any appropriate number of atoms. Usually, the number of atoms is 6 or less.

The purpose of the directing element is to position concurrent bifunctional chemical entities in a consistent manner, e.g. either all horizontally towards the major groove or all vertically towards the major groove. This is achieved through obtaining favourable interactions between major groove atoms of DNA and atoms of the DE and as well between the concurrent DE's. In addition, by use of various substituents, steric hindrance of one but not the other reaction can be achieved. The DE is preferably comprised of a substituted or unsubstituted 5-, 6-, or 7-membered aromatic/heteroaromatic ring system, characterised by potentially having a stacking effect. Counting the shortest distance between SE and BE attachment points, the DE usually has a minimum length of 3 atoms and a maximum length of 6 atoms. In cases where the DE is attached as a substituent at the SE or at the BE, the typical length is 3 to 6 atoms plus additional atoms from potential ring substituents. If the DE is attached at the outer end of the FE, the link should be through a specifically cleavable traceless construction; typically an orto-nitrobenzyl unit attached an amine.

The total linker length (i.e. without the size of the bifunctional entity) is in general from 3 atoms and up to 11 atoms. Combining the different elements in the most efficient way results in a total length (including the bifunctional entity) of between 8 and 15 atoms, resulting in a typical extended length of 8 to 16 Å, and a typical non-extended length of 8-12 Å.

This length is comparable to the dimensions of the DNA double helix which has a total diameter of approximately 18 Å, a cross width of the major groove of approximately 17 Å, and a minimum distance between the linker-attachment of the base and the closest phosphate group of the opposite strand of 12.5 Å. Detailed examples are given in the attached examples.

### Nucleotides

The nucleotides used in the present invention may be linked together in a sequence of nucleotides, i.e. an oligonucleotide. Each nucleotide monomer is normally composed of two parts, namely a nucleobase moiety, and a backbone. The backbone may in some cases be subdivided into a sugar moiety and an internucleoside linker.

The nucleobase moiety may be selected among naturally occurring nucleobases as well as non-naturally occurring nucleobases. Thus, "nucleobase" includes not only the known purine and pyrimidine hetero-cycles, but also heterocyclic analogues and tautomers thereof. Illustrative examples of nucleobases are adenine, guanine, thymine, cytosine, uracil, purine, xanthine, diaminopurine, 8-oxo-N⁶-methyladenine, 7-deazaxanthine, 7-deazaguanine, N⁴,N⁴-ethanocyiosin, N⁶,N⁶-ethano-2,6-diaminopurine, 5-methylcytosine, 5-(C³-C⁶)-alkynylcytosine, 5-fluorouracil, 5-bromouracil, pseudoisocytosine, 2-hydroxy-5-methyl-4-triazolopyridine, isocytosine, isoguanine, inosine and the "non-naturally occurring" nucleobases described in Benner et al., U.S. Pat No. 5,432,272. The term "nudeobase" is intended to cover these examples as well as analogues and tautomers thereof. Especially interesting nucleobases are adenine, guanine, thymine, cytosine, 5-methylcytosine, and uracil, which are considered as the naturally occurring nucleobases.

### Examples of suitable specific pairs of nucleobases are shown below:

### Natural Base Pairs

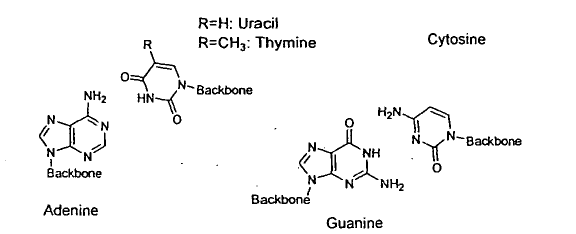

### Synthetic Base Pairs

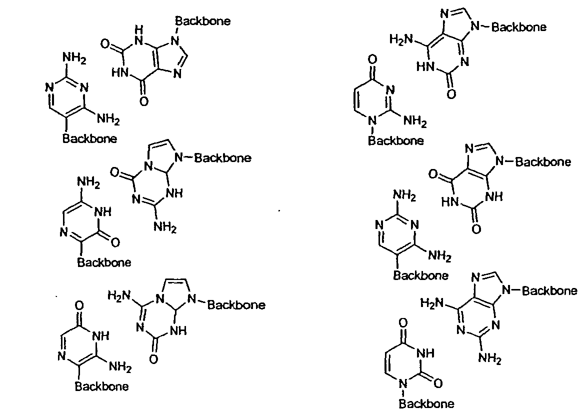

### Synthetic purine bases pairring with natural pyrimidines

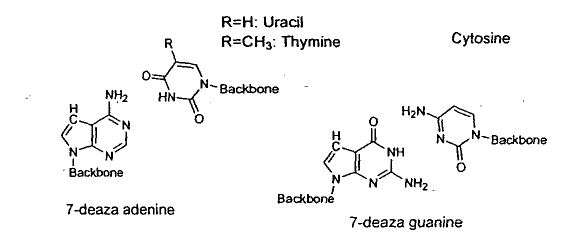

Suitable examples of backbone units are shown below (B denotes a nucleobase):

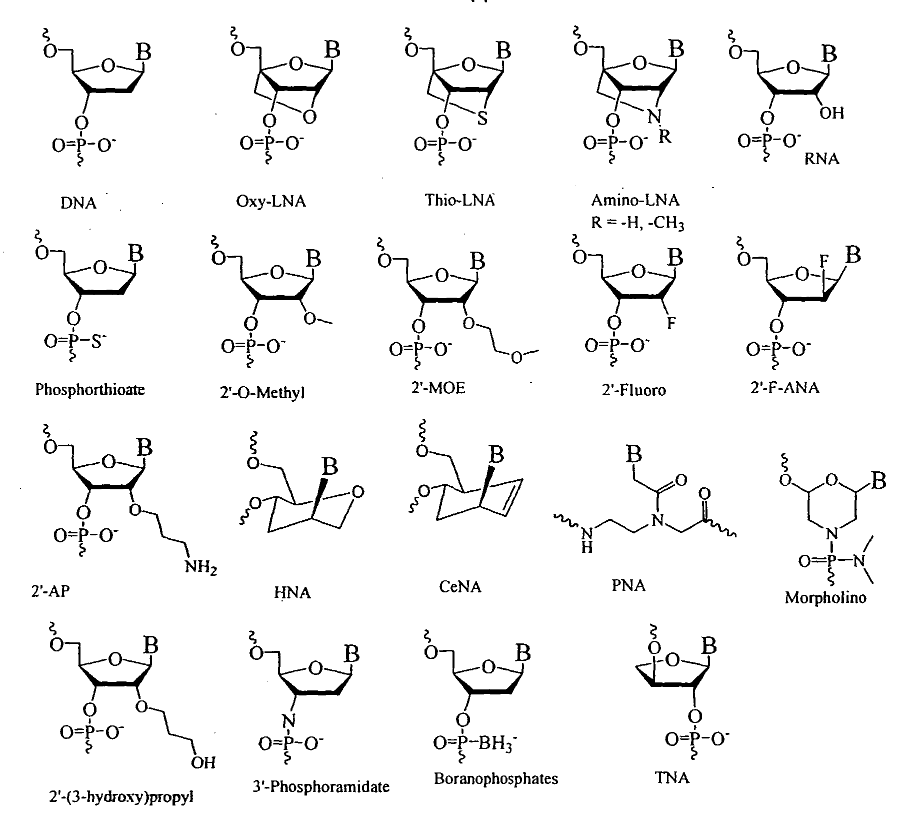

The sugar moiety of the backbone is suitably a pentose but may be the appropriate part of an PNA or a six-membered ring. Suitable examples of possible pentoses include ribose, 2'-deoxyribose, 2'-O-methyl-ribose, 2'-flour-ribose, and 2'-4'-O-methylene-ribose (LNA). Suitably the nucleobase is attached to the 1' position of the pentose entity.

An internucleoside linker connects the 3' end of preceding monomer to a 5' end of a succeeding monomer when the sugar moiety of the backbone is a pentose, like ribose or 2-deoxyribose. The internucleoside linkage may be the natural occurring phospodiester linkage or a derivative thereof. Examples of such derivatives include phosphorothioate, methylphosphonate, phosphoramidate, phosphotriester, and phosphodithioate. Furthermore, the internucleoside linker can be any of a number of non-phosphorous-containing linkers known in the art.

Preferred nucleic acid monomers include naturally occurring nucleosides forming part of the DNA as well as the RNA family connected through phosphodiester linkages. The members of the DNA family include deoxyadenosine, deoxyguanosine, deoxythymidine, and deoxycytidine. The members of the RNA family include adenosine, guanosine, uridine, cytidine, and inosine. Inosine is a non-specific pairing nucleoside and may be used as universal base because inosine can pair nearly isoenergetically with A, T, and C. Other compounds having the same ability of non-specifically base-pairing with natural nucleobases have been formed. Suitable compounds which may be utilized in the present invention includes among others the compounds depicted below

### Examples of Universal Bases:

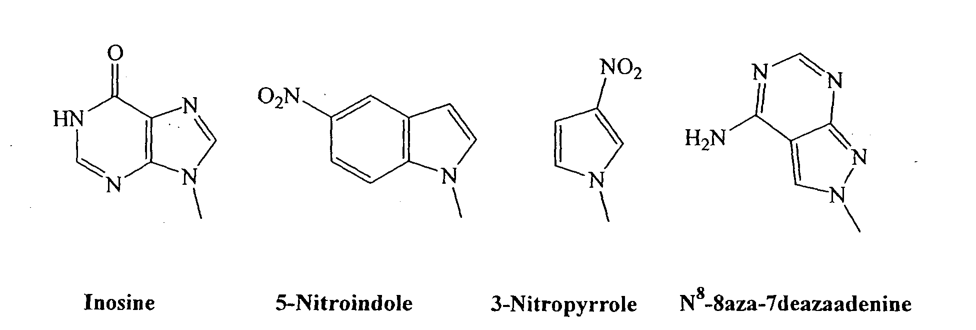

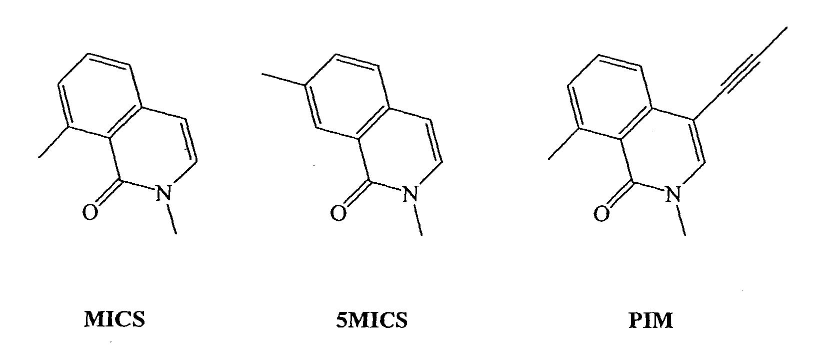

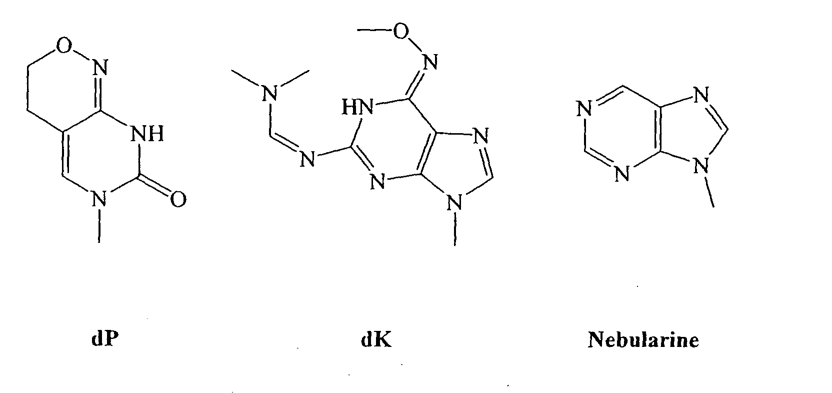

### Examples

### General

Employing chemical entities having two different reacting groups capable of reacting with each other leads to the possibility of two different reactions, either the 3' nucleophile reacts with the 5' electrophile (3'-5' reaction) or the 5' nucleophile reacts with the 3' electrophile (5'-3' reaction). As explained above, the structure of the DNA double helix leads to different geometric nature of the two reactions. By rational linker design this difference can be exploited resulting in building blocks favouring one reaction directionality for the other, resulting in a larger fraction of full-length product and a known polarity.

Computer calculations can provide a measure of the probability of the two reactions. The purpose of these calculations is to analyse various modes of attack for each linker-BE construction, estimating the most probable reaction and thereby the most probable product. Therefore, the conformational space covered by the linker-BE unit and the zones occupied by the reactive groups needs to be estimated.

The conformational space of a specific linker-BE system, i.e. the range of the BE, can be estimated by doing a conformational search. Conformational searches can be performed employing various different software products and within these programs using different searching methods, as is standard knowledge within the field. For systems of the size mentioned herein it is not possible to perform a converged conformational search, that is, to ensure that enough steps have been taken so that the complete potential energy surface has been covered and thereby that the located minimum energy conformation is truly the global minimum for the molecule. However, the purpose of these calculations is to get a picture of the space allowed to be covered by a linker-BE unit and thereby to estimate the most likely approach of attack between two BEs and the possibility for the reacting groups to get within reaction distance. Efficient conformational searching methods employing a rather limited number of steps fulfil this purpose.

By *conformations* are here meant individual structural orientations differing by simple rotation about single bonds. Different conformations may in addition give rise to different overall *configurations,* by which is meant an overall arrangement of the two reactive groups on all modified nucleotides that give rise to one specific direction of reaction.

Referring to Fig. 1, the four linker-BE units arranged with all 'X's' in the same direction corresponds to one specific configuration, and four linker-BE units arranged e.g. with two 'X's' pointing in one direction and the two other in the opposite direction corresponds to another specific configuration. Within one configuration many different conformations are possible, but all of these result in the same 'most probable' product since the overall orientation (direction) of reactive groups is preserved.

The calculations performed in this investigation have employed the MacroModel8.0 software from Schrodinger Inc (MMOD80). Within this program package a series of different searching protocols are available, including the 'Mixed Monte Carlo Multiple Minimum/Low Mode' method (MCMM/LM), shown to be very effective in locating energy minima for large complicated systems.

Having covered the conformational space to a reasonable extent, the structures can be analysed, as follows. It is important to note, that the distances mentioned in the following are reactant distances, i.e. minimum energy distances, and will never be in the range of reaction (i.e. transition state) distances. The possibility therefore exists that the reactant distances for a specific attack seems favourable but that the two groups never can get closer than that, i.e. are incapable of getting within realistic reaction distance. It is therefore necessary to analyse whether the minimum energy structures by simple dihedral rotations will result in structures having the two reacting groups within reaction distance.

For every conformation within a specified energetic interval (e.g. within 50 kJ/mol from the 'global minimum'), the 3'-5' and 5'-3' distances are measured in Å (d3' and d5', respectively) and the energy in kJ/mol. Subtracting 5'-3' from 3'-5' (d3'-d5') gives a measure of the absolute difference in distance between the two reactions. However, since only conformations where one of the two distances is short will lead to reaction it is necessary to divide by the min(d3',d5').

| | | |
|---|---|---|
| Example: | 3' favourable: d3'=3 Å, d5'=7 Å | (d3'-d5')/min(d3',d5')=-1.33 |
| | 5' favourable: d3'=7 Å, d5'=3 Å | (d3'-d5')/min(d3',d5')=1.33 |
| | no reaction: d3'=10 Å, d5'=13 Å | (d3'-d5')/min(d3',d5')=-0.3 |

That is, conformations capable of giving 3' reaction will show by large negative values, conformations leading to 5' reaction by large positive values, and non-reactive conformations will be identified by small positive or negative values.
Plotting these preference values as function of conformational energy and in addition colour code the bars according to min(d3',d5') such that the interesting conformations show in a significant colour leads to a very easy visualization of the linker directionality efficiency.

A favourable linker design with a large preference for only one of the reactions will thus be shown as having either exclusively large negative or large positive values. Small-value conformations do not constitute a problem with respect to cluster formation but will be 'dead' conformations and as a worst case scenario lead to small overall reaction probability. In addition, since the relative weight of a conformation decreases with increasing conformation energy, more significance is placed on the lower energy conformations.

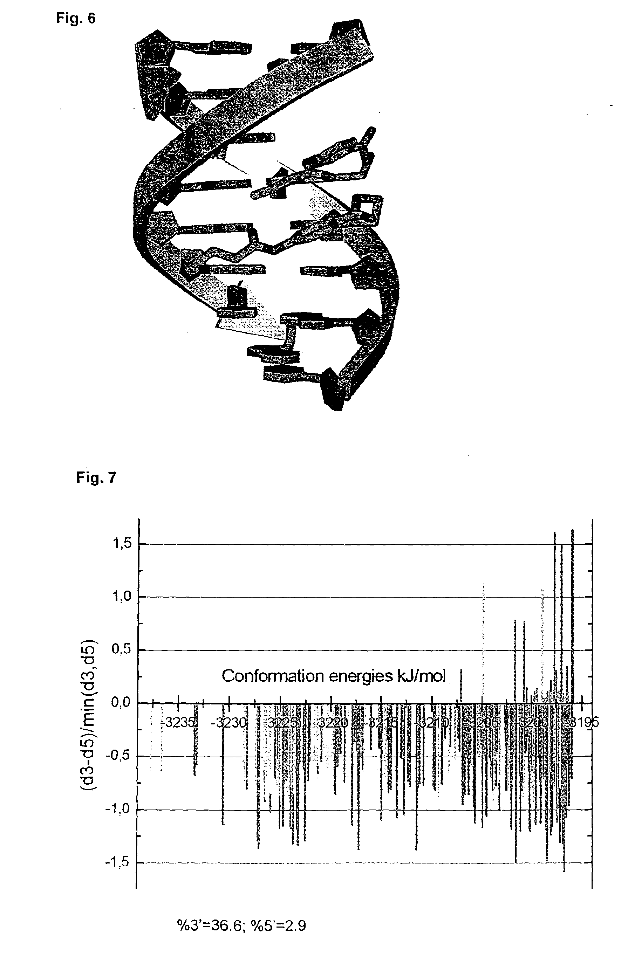

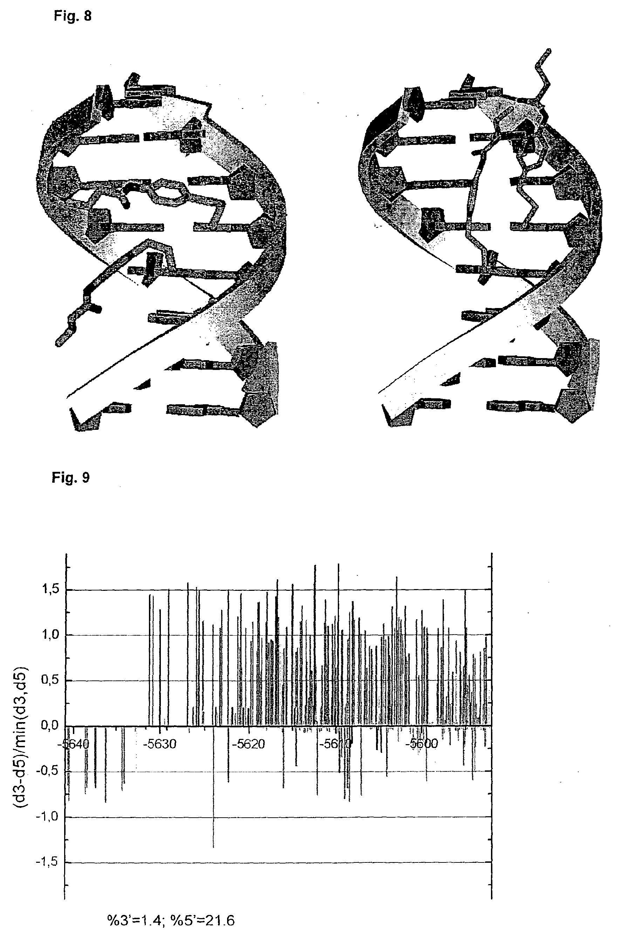

Examples of plots showing linkers leading to a) 3' preference and b) 5' preference are given in Fig. 7 and Fig. 9, respectively. The colour code is grey-scale in these figures, where conformations showing high directionality are shown as dark bars.
Another way of representing the directionality of a bifunctional entity is by numbers describing the percentage of conformations being within a certain tendency towards a specified reaction direction. As an example of a threshold can be used (d3'-d5')/min(d3',d5')<-0,75 indicative of 3' directionality and (d3'-d5')/min(d3',d5')>0,75 indicative of 5' directionality, and the directionality of the linker is then given by %3'=#conf((d3'-d5')/min(d3'.d5')<-0,75)/tota) # conf and %5'=#conf((d3'-d5')/min(d3',d5')>0,75)/total # conf. A favourable linker design will be shown by a large difference between the two numbers. In Figures 7 and 9 the corresponding % directionality number are indicated.

### Example 1. Compound type ND-SE-DE-BE

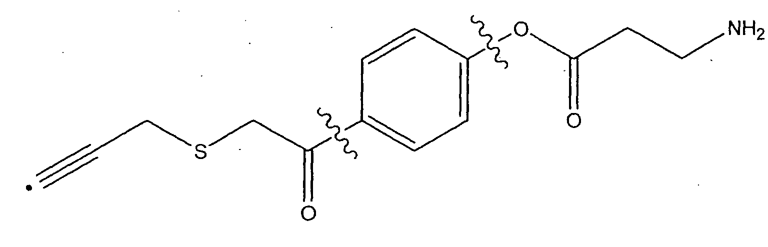

### Computational details

Double-stranded DNA with the base sequence 5'-GCTTTTAG-3' (upper strand) was built using HyperChem7 from HyperCube Inc in the most frequent B-conformation. The linker-BE units were built using ChemDraw Ultra 6.0 and Chem3D Ultra 6.0 from ChemOffice. Linker-BE units and DNA were imported into MMOD80. The linkers were then fused to the two mid nucleotides using the build feature in MMOD80, fusing the methyl carbon atom of the T nucleotides with the appropriate linker atom, in effect creating a modified U nucleotide. In the calculation all DNA atoms were kept frozen, that is, were not allowed to move, in order to decrease the size of the systems and to avoid distortions within the DNA strand. The total system (keeping the DNA atoms frozen) was energy minimised (CONV, arg5=0.05) employing the OPLS-AA force field (FFLD, arg1=11) and the GB/SA water solvation model supplied in MMOD80. Conformational analyses were performed using the MCMM/LM method (LMCS keyword), running 1000 steps (arg 1=1000), exploring a random linear combination of the first 10 modes (arg3=-10), and with a minimum and maximum distance travelled by the fastest moving atom of 3 and 8 Å, respectively (arg7=3, arg8=8). A maximum of 15 torsions (all within the two linker parts of the total system) were allowed to be changed in a MC step (MCNV, arg1=2, arg2=15) and the energy cutoff was 50 kJ/mol (MCSS, arg5=50.0). Extended cut-off distances of 100, 100, and 4 for van der Waal, electrostatics, and hydrogen bonds, respectively were used in all calculations. Finally each conformer was minimized by 500 PR Conjugate Gradient steps. The lowest energy conformation is displayed in Figure 6.

### Results

Running 1000 conformational search steps results in 393 unique conformations with the 'global' minimum located once. The lowest-energy conformation is displayed in Figure 6 and is clearly biased towards 3' reaction direction. The two linkers orient towards the DNA major groove in a regular way, providing stacking of the two directing elements (two benzene rings) and favourable van-der-Waals interactions between linker and DNA atoms. This orientation results in a reaction distance for the 3' attack (amino group of the topmost bifunctional entity (3' end) reacting with the ester group of the lower building block (5' end)) of 5.5 Å as compared to the distance for the 5' attack (amino group of the lower building block (5' end) reacting with the ester group of the upper building block (3' end)) of 9.5 Å. It is important to note here, that these structures are minimum energy structures and thus the distances will never be in transition state range (on the order of 2 Å). However, a transition state leading to a 3' reaction product will be very close both structurally and energetically to the displayed minimum structure, and a linker leading to a minimum structure showing strong 3' preference (i.e. a significantly shorter d3' than d5') is taken as indicative of that linker being capable of leading to 3' directionality also at the transition structure level.

Figure 7 shows the directionality plot of the calculation, according to the reaction distance conversions described in the text above. The plot shows a very clear tendency of this linker to favour a 3' reaction direction, since only very few and high-energy conformations result in positive valued bars. The corresponding %directionality numbers are %3'=36.6 and %5'=2.9.

Thus, use of this linker provides large 3' directionality and in addition, since %3' is a significant high number, a very high reaction probability.

### Example 2. compound type ND-SE-DE-BE

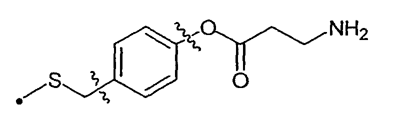

### Computational details

Double-stranded DNA with the base sequence 5'-GCTTTTAG-3' (upper strand) was built using HyperChem7 from HyperCube Inc in the most frequent B-conformation. The linker-FE units were built using ChemDraw Ultra 6.0 and Chem3D Ultra 6.0 from ChemOffice. Linker-FE units and DNA were imported into MMOD80. The linkers were then fused to the two mid nucleotides using the build feature in MMOD80, fusing the methyl carbon atom of the T nucleotides with the appropriate linker atom, in effect creating a modified U nucleotide. In the calculation all DNA atoms were kept frozen, that is, were not allowed to move, in order to decrease the size of the systems and to avoid distortions within the DNA strand. The total system (keeping the DNA atoms frozen) was energy minimised (CONV, arg5=0.05) employing the OPLS-AA force field (FFLD, arg1=11) and the GB/SA water solvation model supplied in MMOD80. Conformational analyses were performed using the MCMM/LM method (LMCS keyword), running 1000 steps (arg 1=1000), exploring a random linear combination of the first 10 modes (arg3=-10), and with a minimum and maximum distance travelled by the fastest moving atom of 3 and 8 Å, respectively (arg7=3, arg8=8). Extended cut-off distances of 100, 100, and 4 for van der Waal, electrostatics, and hydrogen bonds, respectively were used in all calculations. A maximum of 13 torsions (all within the two linker parts of the total system) were allowed to be changed in a MC step (MCNV, arg1=2, arg2=13) and the energy cut-off was 50 kJ/mol (MCSS, arg5=50.0). Finally each conformer was minimized by 500 PR Conjugate Gradient steps. The lowest energy conformation is displayed in Figure 8 left, and a 5' favourable configuration in Figure 8 right.

### Results

Running 1000 conformational search steps results in 566 unique conformations with the 'global' minimum located once. Two low-energy conformations are displayed in Figure 8 left and right, conf 1 and 16 respectively. Conformation 1 has a linker orientation presumably favouring 3' attack, however, the reaction distance is quite long (7.7 Å as compared to 11.7 for 5' attack) and the reaction is unlikely. Conformation 16 shows a 5' biasing linker orientation with a favourable 5' reaction distance (3.6 Å as compared to 8.9 for reaction from 3' direction), well in the range of being close to a reactive transition structure. An overall vertical arrangement for this linker gives a more favourable stacking interaction than a horizontal arrangement and allows for a closer proximity of the linkers and the DNA major groove.

Figure 9 shows the directionality plot of the calculation, according to the reaction distance conversions described above. It can be seen that the first few low-energy conformations are on the borderline of giving rise to 3' directed reactions (negative-valued bars). However, by far the majority of the subsequent conformations are biased towards 5' attack (large positive-valued bars), and the overall appearance of the plot is clearly towards 5' attack. The corresponding %directionality numbers are %3'=1.4 and %5'=21.6. Thus, use of the present linker provides large 5' directionality and in addition, since %5' is a high number, a high reaction probability.

### Example 3. A summary of a series of linkers.

As described in the above examples it is possible by computational analyses to determine the likeliness of a specific linker construction being directional or not. In the table below results are listed for a series of different linkers, demonstrating the broad application of the design rules set up in the present invention. All calculations have been performed as described in Examples 1 and 2.

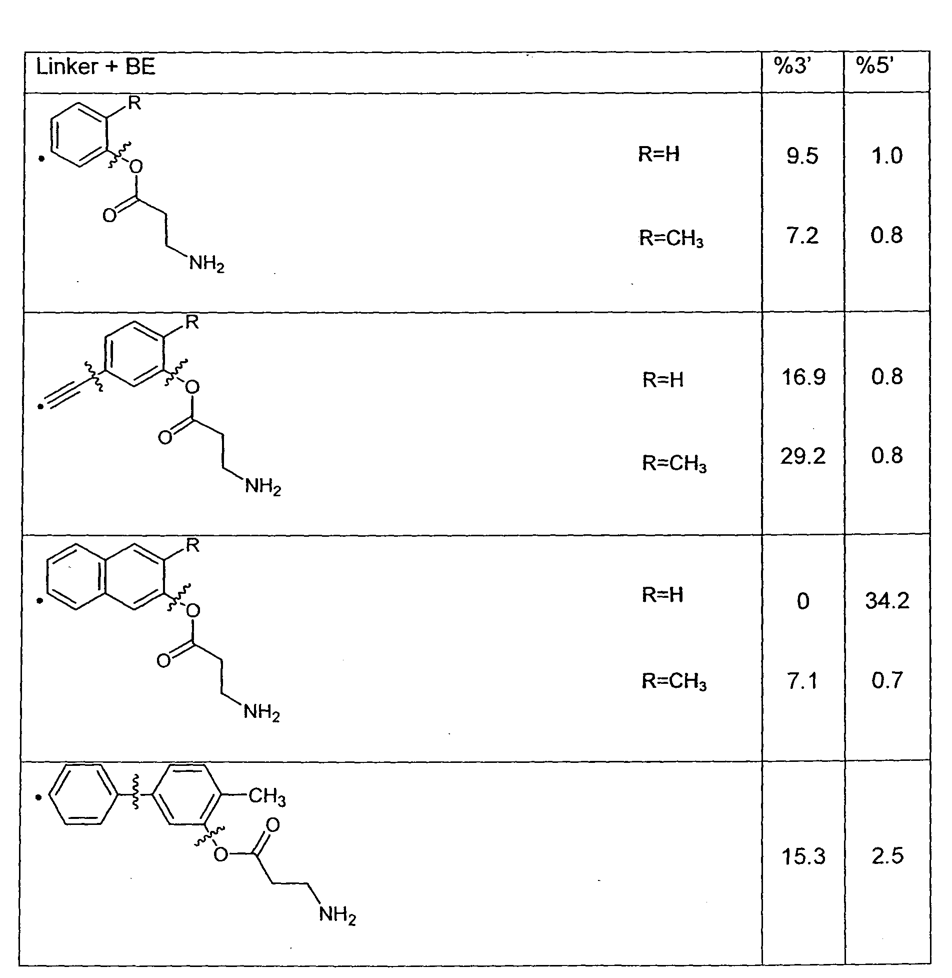

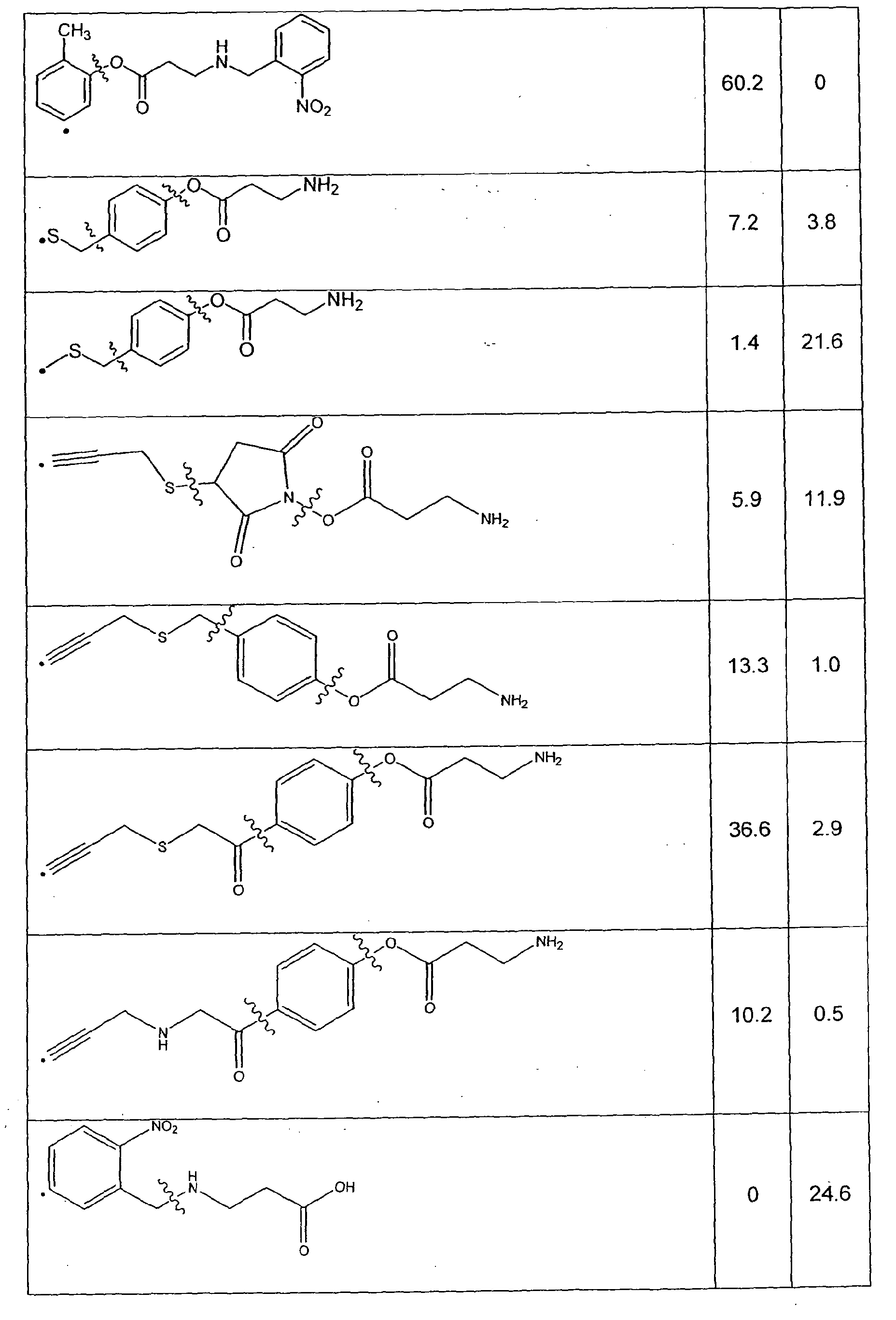

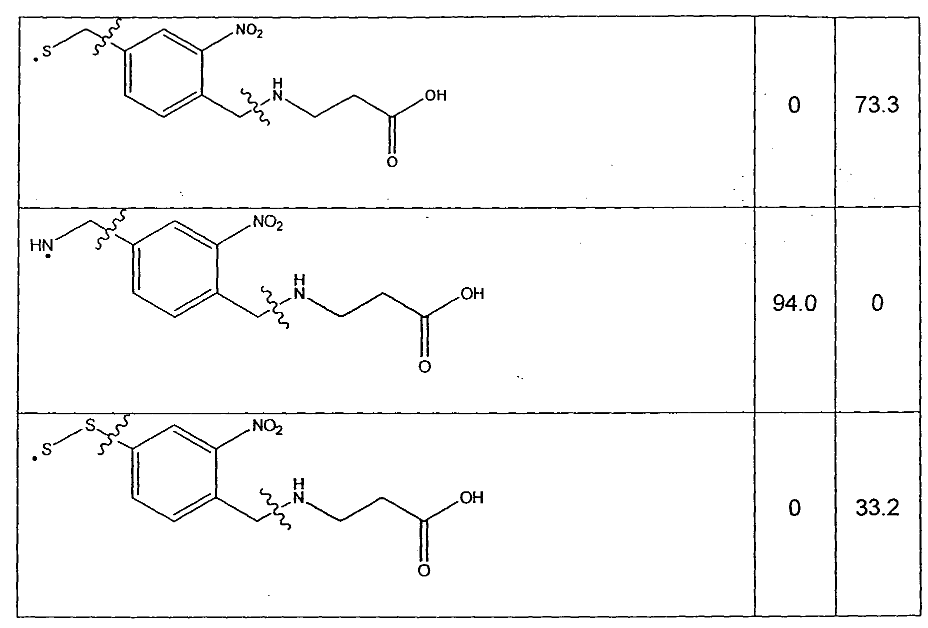

### Example 4: Examples of general linker construction of the type ND-SE-DE-FE

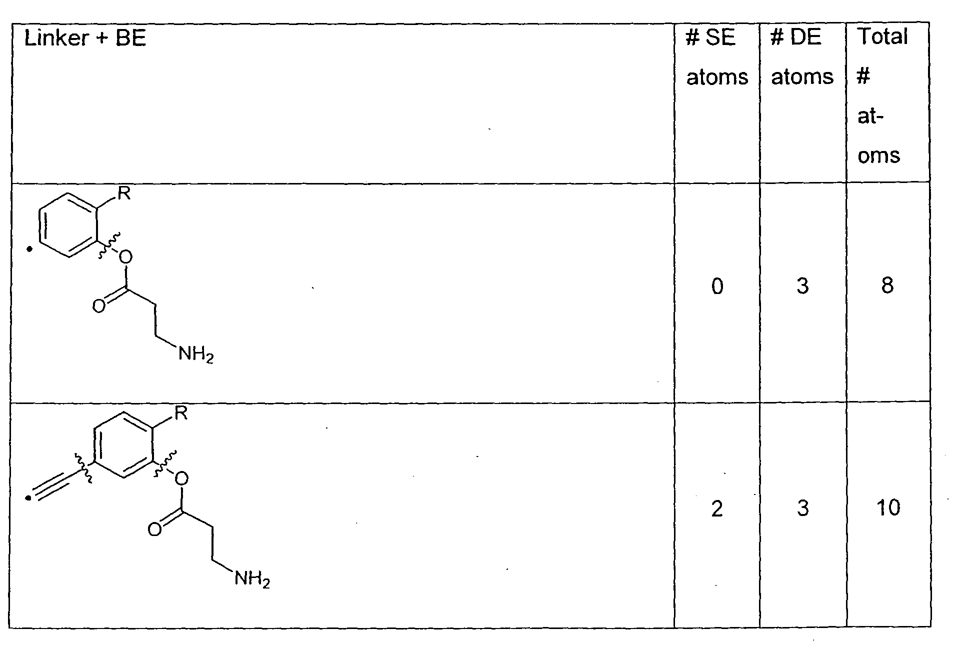

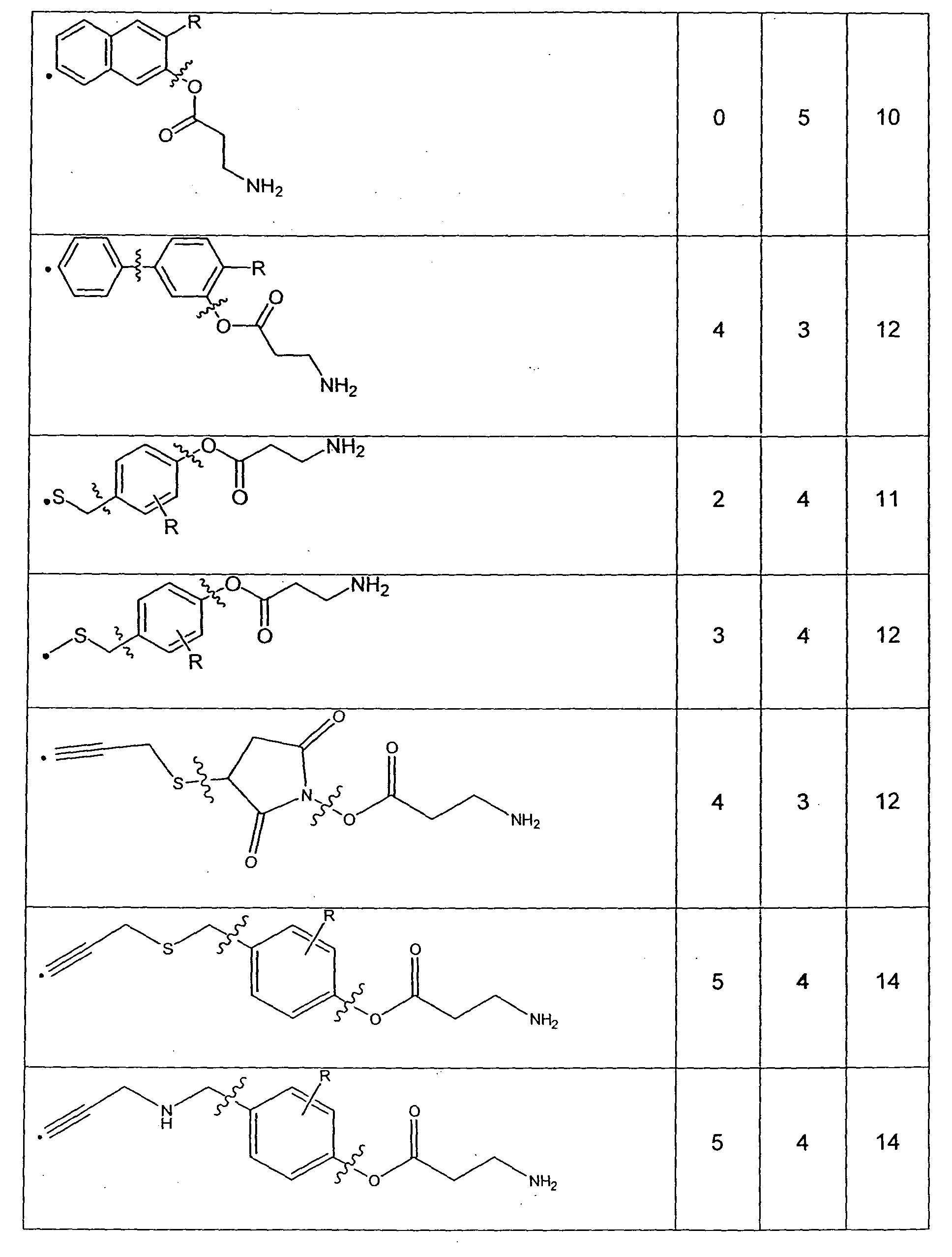

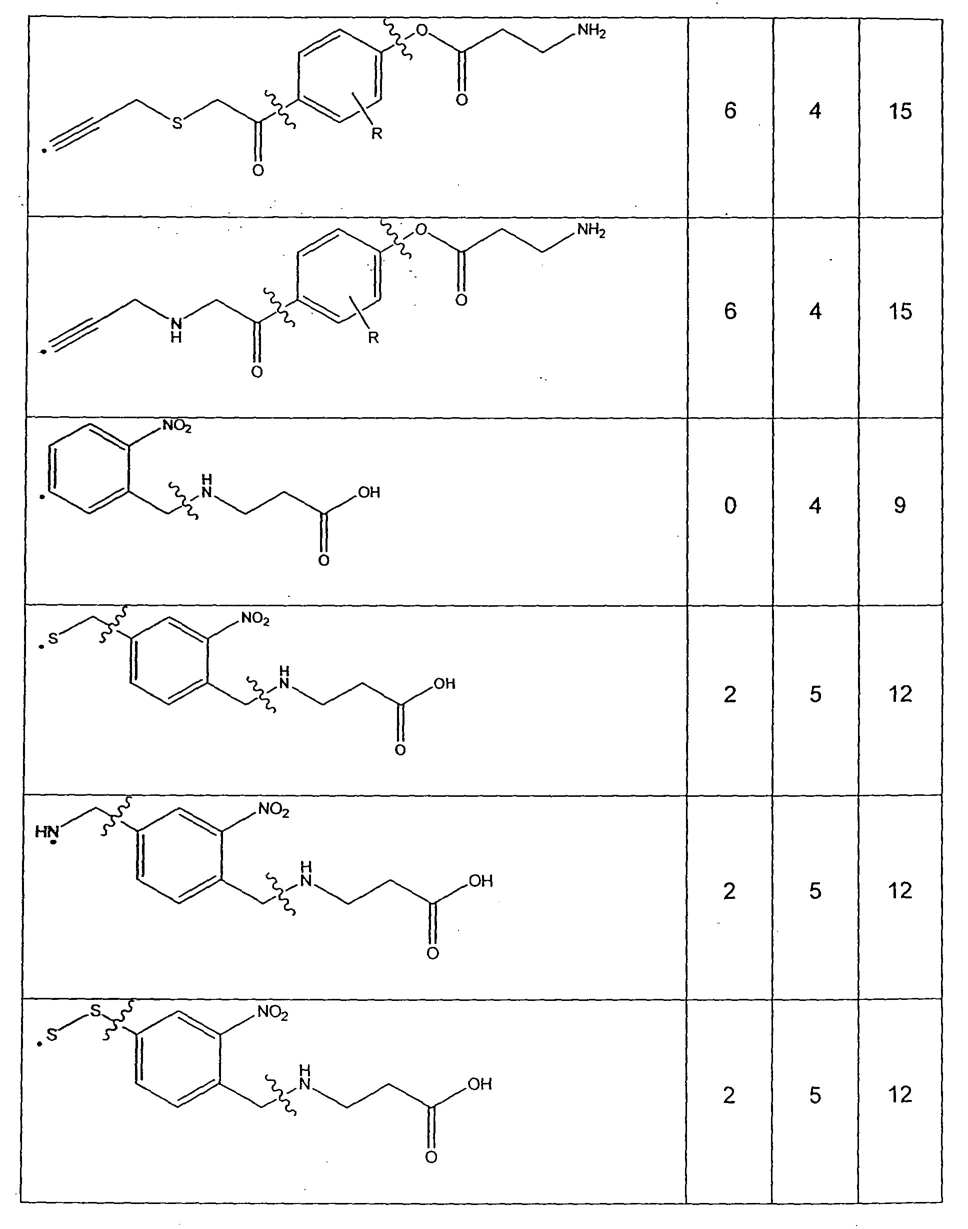

**Example 5**. Examples of general linker constructions of the type

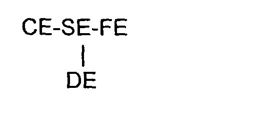

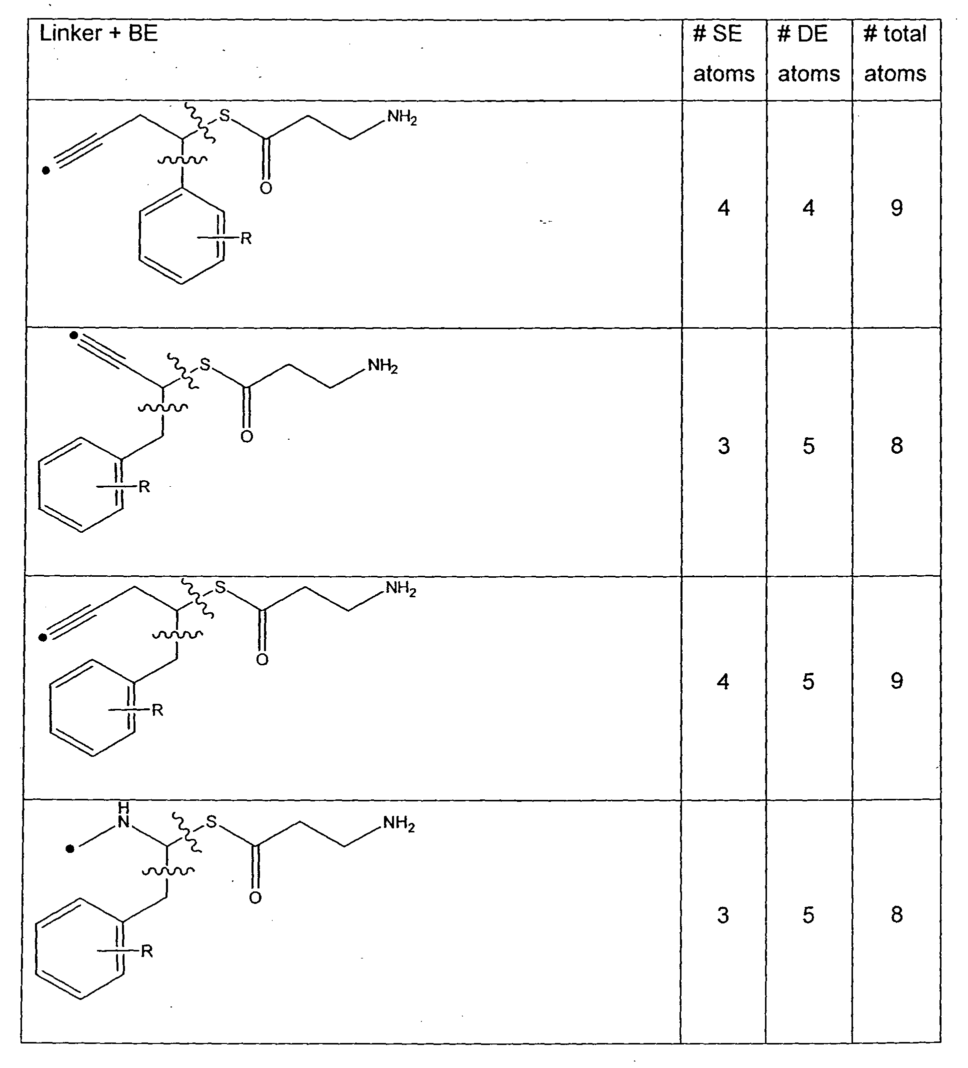

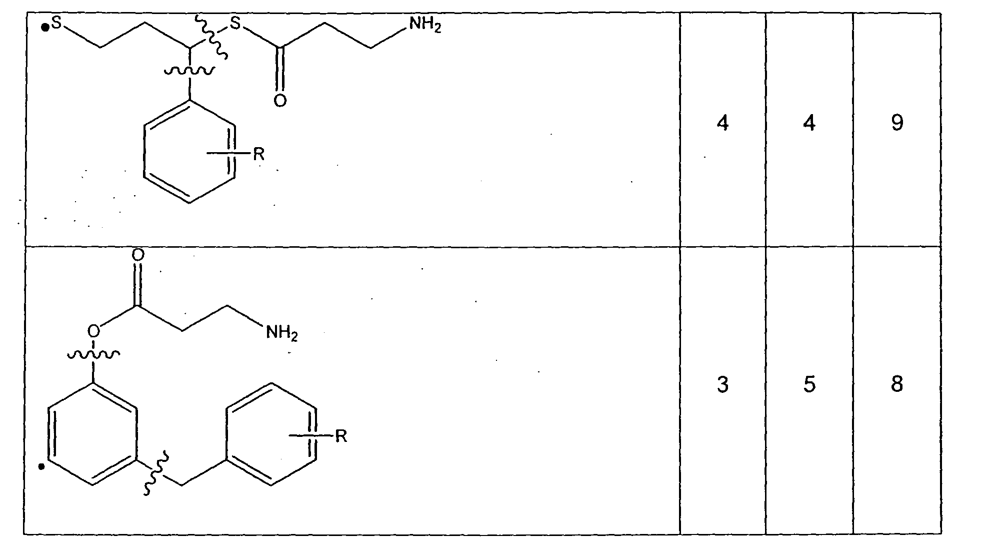

### Example 6. Examples of general linker constructions of the type ND-SE-BE-DE

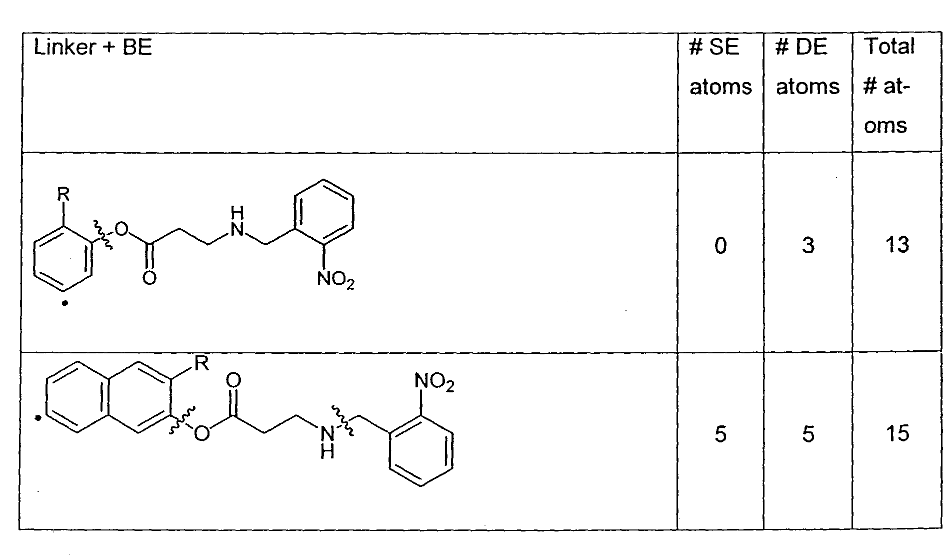

## Claims

1. A compound comprising a bifunctional chemical entity attached to a nucleoside derivative and a directing element capable of positioning the bifunctional chemical entity in a predominate direction.

2. The compound of claim 1, wherein the directing element interacts with one or more major groove atoms or an internucleoside linkage of a double helix nucleic acid.

3. The compound of claim 1 or 2, wherein the directing element comprises an optionally substituted 5-, 6-, or 7-membered ring structure.

4. The compound of claim 3, wherein the directing element comprises an aromatic or hetero-aromatic ring system.

5. The compound of claim 1 to 4, wherein the bifunctional chemical entity comprises a nucleophile and an electrophile as the two functionalities.

6. The compound wherein the functionalities are protected by suitable protection groups.

7. The compound of claim 1 to 6, wherein the bifunctional chemical entity is attached to the nucleoside derivative through a spacing element.

8. The compound of claim 7, wherein the spacing element comprises a chemical bond which includes electrons from overlapping p-orbitals.

9. The compound of claim 7 or 8, wherein the spacing element includes a triple bond, an aromatic- or heteroaromatic ring system, or a hetero atom.

10. The compound of any of the claims 1 to 9, wherein the extended length between the bifunctional chemical entity and the nucleoside is between 3 and 12Å.

11. The compound of any of the preceding claims, wherein the bifunctional chemical entity is attached through a linker to the nucleobase of the nucleoside.

12. The compound of claim 11, wherein the nucleobase is selected among adenine, 7-deaza-adenine, uracil, guanidine, 7-deaza-guanidine, thymine, and cytosine.

13. The compound according to any of the preceding claims, wherein the bifunctional chemical entity is attached through a linker to the 5 position of pyrimidine type nucleobases or the 7 or 8 position of purine type nucleobases.

14. The compound according to any of the preceding claims, wherein the extended length between the directing element and the bifunctional chemical entity is 4Å or less.

15. The compound according to claim 14, wherein the directing element is attracted or repulsed by major groove or internucleoside linkage atoms of a nucleic acid double helix.

16. A nucleotide analogue comprising the compound according to any of the claims 1 to 15.

17. The nucleotide analogue of claim 16 being a substrate for a polymerase.

18. An oligonucleotide analogue comprising the compound according to any of the claims 1 to 15.

19. The oligonucleotide according to claim 18, being a substrate for a ligase.

20. A method for directing the structural orientation of a bifunctional chemical entity, wherein a nucleoside derivative comprising a bifunctional chemical entity further comprises a directing element capable of positioning the bifunctional chemical entity in a predominate direction.

21. The method of claim 20, wherein the directing element interacts with the major groove atoms or internucleoside linkage of a double helix nucleic acid.

22. The method of claim 20 or 21, wherein the directing element comprises an optionally substituted 5-, 6-, or 7-membered ring structure.

23. The method of claims 20 to 22, wherein the directing element comprises an aromatic or hetero-aromatic ring system.

24. A method for obtaining an encoded molecule comprising
Contacting a template nucleic acid with one or a plurality of compounds according to any of the claims 1 to 19, under conditions that provides for the formation of a complementary template,
Reacting the bifunctional chemical entities to form an encoded molecule.

25. The method according to claim 24, wherein the template comprises an oligonucleotide and the conditions providing for the formation of a complementary template include a polymerase or a ligase.

26. The method according to claim 24 or 25, wherein the compound is comprised in a nucleotide triphosphate.

27. A polymer obtainable by the method according to any of the claims 24 to 26.

28. A double stranded nucleic acid having one or more compounds according to any of the claims 1 to 18 incorporated therein.

29. The double stranded nucleic acid according to claim 28, wherein the bifunctional chemical entities have been reacted under conditions in which they had a predominate orientation.

30. The double stranded nucleic acid according to claim 28 or 29, wherein one or more linking moieties have been cleaved to display the encoded molecule.
